**Europäisches Patentamt**

**European Patent Office**

**Office européen des brevets**

(11) Veröffentlichungsnummer: **0 200 155 B1**

(12) **EUROPÄISCHE PATENTSCHRIFT**

(45) Veröffentlichungstag der Patentschrift: **08.07.92**

(51) Int. Cl.5: **A61K 33/40**

(21) Anmeldenummer: **86105646.3**

(22) Anmeldetag: **24.04.86**

(54) **Wässrige Chloritmatrix-Lösung.**

(30) Priorität: **02.05.85 DE 3515745**

(43) Veröffentlichungstag der Anmeldung:
**05.11.86 Patentblatt 86/45**

(45) Bekanntmachung des Hinweises auf die
Patenterteilung:
**08.07.92 Patentblatt 92/28**

(84) Benannte Vertragsstaaten:
**AT BE CH DE FR GB IT LI LU NL SE**

(56) Entgegenhaltungen:
**EP-A- 0 093 875**
**US-A- 4 086 333**
**US-A- 4 296 102**
**US-A- 4 296 103**

**UNLISTED DRUGS, Band 36, Nr. 3, März 1984,
Seite 51, New York, US; "Oxoferin"**

(73) Patentinhaber: **OXO Chemie GmbH
Im Neuenheimer Feld 517
W-6900 Heidelberg(DE)**

(72) Erfinder: **Kühne, Friedrich W., Dr.
Bergstrasse 72
W-6900 Heidelberg(DE)**

(74) Vertreter: **Grussdorf, Jürgen, Dr. et al
Patentanwälte Zellentin & Partner Rubensstrasse 30
W-6700 Ludwigshafen(DE)**

## Beschreibung

Die Erfindung betrifft eine wässrige Lösung einer chemisch stabilisierten Chloritmatrix zur intravenösen und perioperativen Verabreichung.

Stabilisierte Chloritmatrices, die aktivierten Sauerstoff enthalten, sind aus der DE-OS 32 13 389 bekannt und sie werden in Form wässriger Lösungen mit einer Konzentration von 10,4 µg Chloritkomplex pro ml Lösung (0,14 µMol/ml) als Wundbehandlungsmittel bereits erfolgreich eingesetzt (Unlisted drugs, 36, 1985, S. 51).

Weiterhin ist aus USP 4296103 bekannt, eine mit Perborat stabilisierte Chloritmatrix als antiseptische Mischung oral zu applizieren. Diese wässrigen Lösungen sollen ca. 90 - 130 mg/ml Chlorit (ca. 1 - 2 µMol/ml) enthalten.

Versuche, solche bei Wundbehandlungen verwendeten Chloritmatrix-Lösungen bei infizierten Versuchstieren intravenös oder durch Tropfinfusion zu verabreichen, scheiterten, da keinerlei therapeutische, sonderneher toxische Effekte zu beobachten waren. Ein Einsatz der Lösungen auf diesem Wege schien ausgeschlossen.

Ganz überraschend wurde nun gefunden, dass entgegen der herrschenden Meinung, Lösungen einer stabilisierten Chloritmatrix in einem ganz bestimmten Konzentrationsbereich pro kg Körpergewicht des behandelten Menschen oder Tieres auch intravenös und perioperativ verabreicht werden können, da bei der bestimmten Dosierung keine toxische, sondern eine effektive therapeutische Wirkung auftritt.

Es eignet sich eine wässrige Lösung Lösung einer chemisch stabilisierten Chloritmatrix zur intravenösen und perioperativen Verabreichung in einer dosierten Menge von $6,2 \times 10^{-6}$ Mol $ClO_2^-$ bis zu $9,3 \times 10^{-5}$ Mol $ClO_2^-$ pro kg Körpergewicht bei Menschen und Tieren.

Die Lösung enthält die Chloritmatrix in einer Konzentration von 12 bis 72 µMol $ClO_2^-$/ml.

Die positive Wirkung einer solchen Lösung wurde an infizierten Versuchstieren festgestellt. Bei Versuchen wurde mit therapeutischen Mengen von 0,60 bis 60 µMol $ClO_2^-$/kg Körpergewicht gearbeitet, die in Form einer gebrauchsfertigen Lösung verabreicht wurden.

Versuchsergebnisse:

Verwendet wurde eine wässrige isotone Lösung einer Matrix aus Chloritionen, mit darin eingeschlossenem stabilisiertem aktivierfem Sauerstoff. Die Prüfsubstanz enthielt eine Konzentration von 60 µMol/l, bezogen auf Chlorit.

Die Einstellung der Dosis erfolgte mit steriler phys. NaCl-Lösung.

Versuchstiere:

Männliche Balb/cABOM (Bomholtgård, Ry, Dänemark) von 20 ± 1 g Körpergewicht.

n = 30 Tiere pro Gruppe.

Die experimentelle Infektion erfolgte mit Candida albicans ATCC 10231. Die Mikroorganismen wurden in flüssigem Medium gezüchtet, aus der log. Wachstumsphase entnommen, gewaschen und nach mikroskopischer Zählung mit steriler phys. NaCl-Lösung auf eine Dichte von $6 \times 10^6$ Zellen/0,25 ml, entsprechend der in Vorversuchen ermittelten ID 75, aufgenommen. Die experimentelle Infektion erfolgte durch i.v. Injektion dieser Dosis.

Die Therapie der Tiere erfolgte durch einmalige i.v. Injektion der Testlösung 1 h nach der Infektion. Hierzu wurden 2 Gruppen mit verschiedenen Dosen der Prüfsubstanz angesetzt (phys. NaCl-Lösung für die Kontrollen).

Eine Gruppe erhielt jeweils in 0,5 ml 24,64 µg und eine andere Gruppe 147,8 µg der Prüfsubstanz, entsprechend 0,20 ml der Ausgangslösung pro kg = 1,232 mg/kg bzw. 1,20 ml der Prüfsubstanz pro kg = 7,392 mg/kg.

Vorversuche hatten ergeben, daß die einmalige Injektion dieser Dosen in die Schwanzvenen der Tiere keine Nekrosen verursachte.

Die Infektionsversuche ergaben, daß die einmalige Applikation der kleineren Dosis zu einer eindeutigen Verbesserung der Überlebenschancen bei dieser experimentellen Infektion führt, die einmalige Applikation der höheren Dosis aber ebenso eindeutig zu einer Verschlechterung. Dies ist sowohl aus dem Verlauf der Mortalitätsrate als auch an der Zahl der überlebenden Tiere in den drei Gruppen zu ersehen.

Die Ergebnisse sind in der beigefügten graphischen Darstellung verdeutlicht, wobei die Sterblichkeitsrate und Überlebensrate nach 11 Tagen angegeben ist. Die unbehandelten Versuchstiere (Kontrollversuche) zeigten eine Überlebensrate von 23,3%, diejenigen mit einer 0,2 µg/0,5 ml Chloritmatrix-Lösung behandelten Versuchstiere zeigten eine Überlebensrate von 50%, während die 1,2 µg/0,5 ml Chloritmatrix-Lösung mit einer Überlebensrate von 6,7 sich als überaus toxisch erwies. Die positive Wirkung einer 0,2 µg/0,5 ml Lösung ist unerwartet hoch, während eine 1,2 µg/0,5 ml Lösung die bisherige Meinung der Fachwelt deutlich bestätigt.

Laufende Versuche zeigen ferner, dass auch andere als mit Sauerstoff stabilisierte Chloritmatrix-Lösungen verwendet werden können. Die Chloritmatrixionen erweisen sich auch in anderer als mit Sauerstoff stabilisierter Form für entsprechende intravenöse und perioperative Anwendung geeignet.

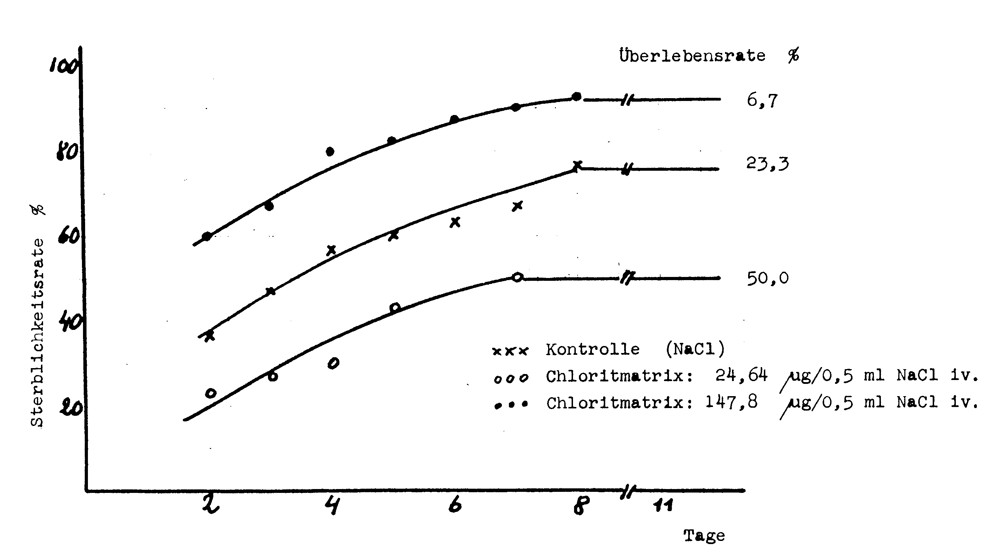

**Patentansprüche**

**1.** Wässrige Lösung einer chemisch stabilisierten Chloritmatrix zur intravenösen und perioperativen Verabreichung in einer dosierten Menge von 6,2 x 10 $^{-6}$ Mol $ClO_2^-$ bis zu 9,3 x 10$^{-5}$ Mol $ClO_2^-$ pro kg Körpergewicht bei Menschen und Tieren, **dadurch gekennzeichnet,** daß darin die chemisch stabilisierte Chloritmatrix in einer Konzentration von 12 bis 72 $\mu$Mol $ClO_2^-$/ml enthalten ist.

**Claims**

**1.** Aqueous solution of a chemically stabilised chlorite matrix for intravenous and perioperative administration in a dosed amount of 6.2 x 10$^{-6}$ Mol $ClO_2{}^-$ to 9.3 x 10$^{-5}$ Mol $ClO_2{}^-$ per kg of body weight in humans and animals, characterised in that the chemically stabilised chlorite matrix is contained therein in a concentration of 12 to 72 $\mu$Mol $ClO_2{}^-$/ml.

**Revendications**

**1.** Solution aqueuse d'une matrice de chlorite chimiquement stabilisée pour une administration intraveineuse et périopératoire en une quantité dosée de 6,2 x 10$^{-6}$ moles de $ClO_2{}^-$ jusqu'à 9,3 x 10$^{-5}$ moles de $ClO_2{}^-$ par kg de poids de corps chez les êtres humains et les animaux, caractérisée en ce que la matrice de chlorite chimiquement stabilisée est contenue dedans en une concentration de 12 à 72 $\mu$moles de $ClO_2{}^-$/ml.